(19) Europäisches Patentamt
European Patent Office
Office européen des brevets

(11) Publication number: **0 089 426**
**B1**

# (12) EUROPEAN PATENT SPECIFICATION

(45) Date of publication of patent specification: **11.06.86**

(51) Int. Cl.[4]: **C 07 C 101/447,** A 61 K 31/195, A 61 K 31/22 // C07D209/34

(21) Application number: **82301438.6**

(22) Date of filing: **19.03.82**

## (54) 2-Amino-6-biphenylacetic acids.

(43) Date of publication of application:
**28.09.83 Bulletin 83/39**

(45) Publication of the grant of the patent:
**11.06.86 Bulletin 86/24**

(84) Designated Contracting States:
**AT BE CH DE FR GB IT LI LU NL SE**

(56) References cited:
**DE-A-1 518 528**
**DE-A-2 355 084**
**US-A-4 045 576**

(73) Proprietor: **A.H. ROBINS COMPANY, INCORPORATED**
**1407 Cummings Drive P.O. Box 26609**
**Richmond Virginia 23261 (US)**

(72) Inventor: **Walsh, David Allan**
**9504 Lakewater Court**
**Richmond Virginia 23229 (US)**

(74) Representative: **Kearney, Kevin David Nicholas et al**
**KILBURN & STRODE 30 John Street**
**London, WC1N 2DD (GB)**

**EP 0 089 426 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Technical Field

The present invention relates to novel biphenylacetic acids and is more particularly concerned with certain 2-amino-6-biphenylacetic acids, esters, and salts for example metal salts, compositions thereof, methods for the production thereof and use of the same.

Background Art

Various biphenylacetic acids have been demonstrated to possess anti-inflammatory activity as disclosed in U.S. Patent 3,784,704. Biphenylacetic acids are also disclosed in US Patent 3,966,978. Biphenylacetic acids and esters are further disclosed in German Offenlegungsschrift 1518528. German Offenlegungsschrift 2,355,084 discloses methyl 3-amino-4-biphenylacetate as an intermediate in the preparation of phenanthridones.

Disclosure of the Invention

According to one aspect of the present invention there is provided a compound having the structural formula:

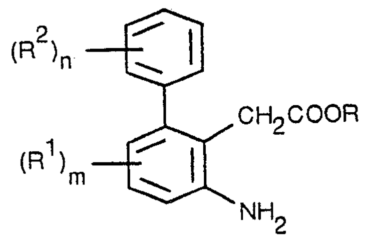

Formula I

wherein:

R represents a hydrogen atom or a lower alkyl group,

$R^1$ represents fluorine, chlorine, or bromine atom or a lower alkyl group or an amino group;

$R^2$ represents a lower alkyl, lower alkoxy, amino, or trifluoromethyl group or a fluorine, chlorine or bromine atom;

n is 0—3 and m is 0—3,

and the pharmaceutically acceptable salts thereof or a hydrate thereof.

Preferred compounds include those in which $R^1$ represents a methyl group, an amino group or a chlorine atom, and m = 1 and n = 0; those in which m = 0, $R^2$ represents a methoxy group, an amino group or a chlorine atom and n = 1; and those in which m = 0, $R^2$ represents a methoxy group or a bromine atom and n = 2.

In another aspect of the present invention there is provided a therapeutic composition suitable for providing muscle relaxation comprising (a) an effective amount of a compound having the structural formula of Formula I and (b) a pharmaceutically acceptable carrier therefor.

The present invention also extends to compounds of Formula I for use in providing muscle relaxation in a living animal body with a minimum of undesirable side effects by internally administering to the said living animal body an effective amount of the said compound.

In still yet another aspect of the present invention there is provided a therapeutic composition suitable for alleviating inflammation comprising (a) an effective amount of a compound having the structural formula of Formula I, and (b) a pharmaceutically acceptable carrier therefor.

The present invention also extends to compounds of Formula I for use in alleviating inflammation in a living animal body with a minimum of undesirable side effects by internally administering to the said living animal body an effective amount of the said compound.

The novel compounds of this invention are useful as muscle relaxant and anti-inflammatory agents. The compounds may be administered alone or with suitable pharmaceutical carriers to warm blooded animals, mammals such as felines, canines and humans and can be in solid or liquid form as, for example, tablets, capsules, powders, solutions, suspensions or emulsions.

The compounds can be administered orally, parenterally, subcutaneously or intramuscularly. The unit dosage form can contain from about 1 to about 100 milligrams of a compound of this invention and can be administered in multiples thereof up to a daily dosage level of about 500 milligrams.

The solid unit dosage form can be a gelatin capsule containing a compound of this invention and a pharmaceutically acceptable filler or carrier such as sucrose, lactose or corn starch. Tablets containing the compounds represent another embodiment of this invention and may be prepared using conventional tableting materials.

The novel concept of the present invention resides in the provision of therapeutically active 6-biphenylacetic acids, esters and salts, for example metal salts, which have a primary amino group ortho to the acetic acid group. Therapeutically active compounds possessing such an arrangement have been heretofore unknown prior to the present invention.

The anti-inflammatory utility of the compounds of this invention was determined using a modification of the Evans Blue-Carrageenan Pleural Effusion Assay of Sancilio, L. F., J. Pharmacol, Exp. Ther. *168*, 199—204 (1969).

The muscle relaxant utility of the compounds of the present invention was determined using the loss of righting test as set forth by A. P. Roszkowski, *J. Pharmacol. and Expt. Therapeutics*, Vol 192, page 75 (1960).

It has been surprisingly and unexpectedly found that the 2-amino-6-biphenylacetic acids of the present invention exhibit significant muscle relaxant activity, while 2-aminobiphenylacetic acids having the phenyl substituent in the 3 and 5 positions exhibit no such activity. The surprising muscle relaxant properties of the 2-amino-6-biphenylacetic acids is clearly shown in the following Table.

TABLE I

| Substituent | Dose (mg/kg) | Percent loss of Righting reflex |
|---|---|---|
| 3-phenyl | 250 | 0 |
| 5-phenyl | 100 | 0 |
| 6-phenyl | 100 | 100 |
| 6-phenyl | 50 | 0 |

In the definition of symbols in the formulae hereof and where they appear elsewhere throughout this specification, the terms having the following significance.

The term "lower alkyl" as used herein means straight and branched chain radicals of up to six carbon atoms inclusive, preferably no more than four carbon atoms, and is exemplified by such groups as methyl, ethyl, propyl, isopropyl, butyl, sec. butyl, tertiary butyl, amyl, isoamyl and hexyl. The term "lower alkoxy" has the formula —O-lower alkyl.

When m = 2 or n = 2 or 3, $R^1$ or $R^2$ can be the same or different radicals.

Illustrative of pharmaceutically acceptable salts are the sodium, potassium, calcium, magnesium, zinc, and copper salts and the hydrates thereof.

The compounds of Formula I wherein R represents hydrogen may be prepared by hydrolysis of 4-phenylindolin-2-ones (II) in aqueous basic solution followed by neutralization of the basic reaction mixture with a suitable organic acid such as acetic acid or a dilute acid as indicated by the following reaction scheme:

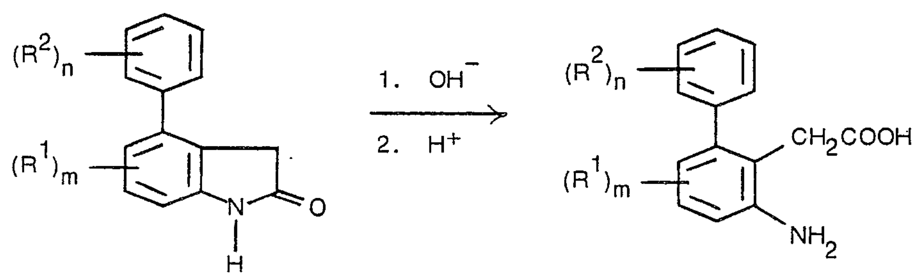


Formula II

wherein $R^1$ and $R^2$ are as defined hereinabove.

The hydrolysis of a 4-phenylindolin-2-one (II) may be carried out in a dilute aqueous base as, for example, a 3N sodium hydroxide solution, for a period of from about 1.0 to about 60 hours, usually until the indolin-2-one has dissolved. The hydrolysis may be carried out in an inert atmosphere using nitrogen. The hydrolysis mixture may be filtered to remove base-insoluble materials and the pH of the basic solution is adjusted to a pH of from 6—7 by the addition of a weak organic acid such as glacial acetic acid or a dilute mineral acid such as hydrochloric acid.

The free acids can contain water of crystallization. Therefore the free acids containing various degrees of hydration are included within the scope of the present invention.

The lower alkyl esters of Formula I are preferably prepared from the acids which are converted to an alkali metal salt, preferably the sodium or potassium salt which is isolated, dried, and then reacted in a suitable solvent as for example, dimethyl formamide, with an alkyl halide, preferably an alkyl iodide, to furnish the desired ester.

The 4-phenylindolin-2-ones (II) may be prepared from appropriately substituted biphenylamines (V) by the following reaction sequence, wherein $R^1$ and $R^2$ have the values hereinabove defined except

additionally $R^1$ and $R^2$ may be nitro in Formulas III, IV and V, and R represents a lower alkyl group, preferably an ethyl group. The reaction conditions employed are more fully set forth hereinafter in the specific preparations which follow.

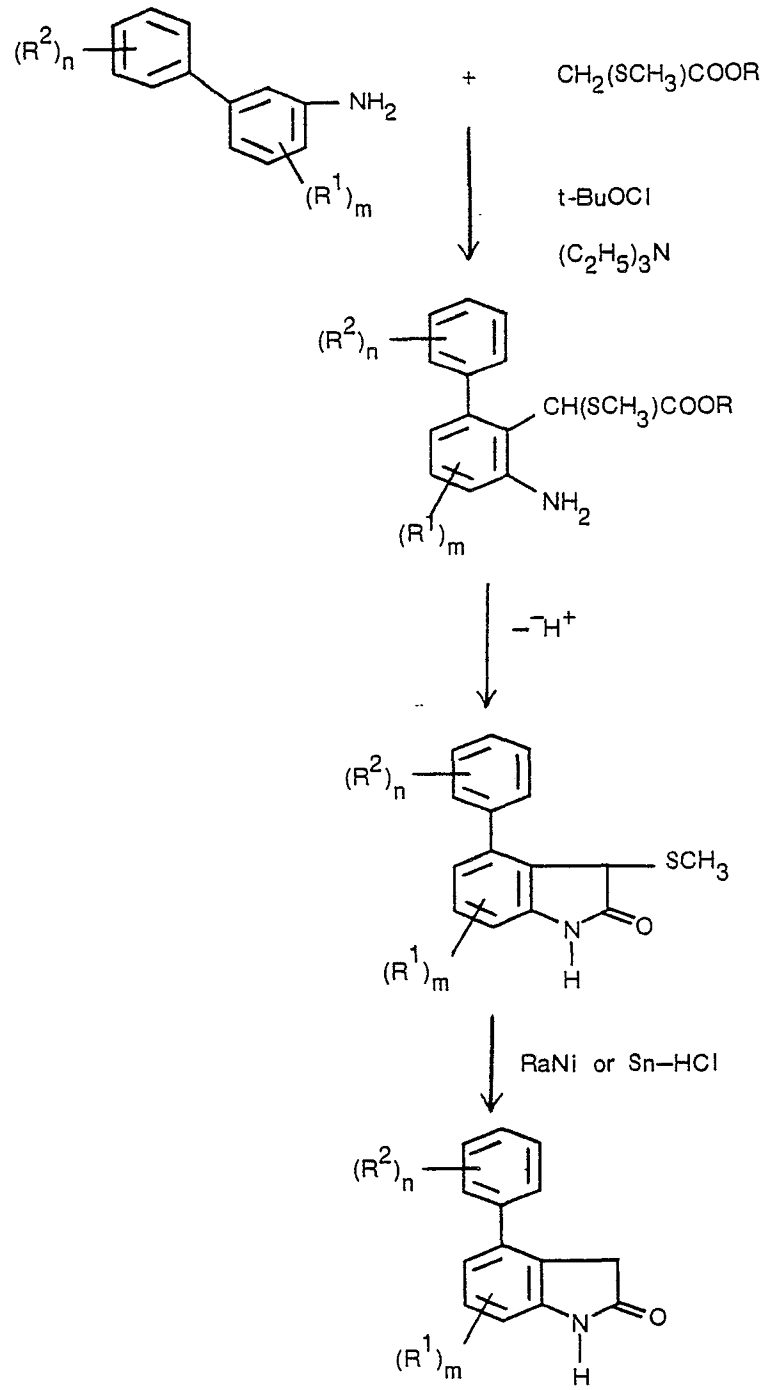


Formula V

Formula IV

Formula III

Formula II

Compounds of Formula II wherein $R^1$ or $R^2$ represent an $—NH_2$ group may be prepared from the corresponding biphenyl amine wherein $R^1$ or $R^2$ represent an $—NO_2$ group, the resulting $—NO_2$ radical in Formula III being reduced by RaNi (Raney Nickel) or Sn/HCl at the same time as the methylthio group is removed. Additional reducing agent may be required and in the instance of tin, three additional moles per mole of $—NO_2$ are required as well as sufficient HCl to maintain acid conditions.

Best Mode of Carrying out the Invention

The invention may be put into practice in various ways and a number of specific embodiments will be described to illustrate the invention with reference to the accompanying examples which are preceeded by preparations which describe the preparation of starting materials and intermediates for use in the examples.

*Preparation 1*

3-Methylthio-4-phenylindolin-2-one

m-Biphenylamine hydrochloride (35.3 g, 0.172 mole) was partitioned between 300 ml of methylene chloride and 200 ml of 5% sodium hydroxide solution. The layers were separated and the methylene chloride layer was dried (sodium sulphate) and its volume adjusted to 400 ml with additional methylene chloride. The solution was cooled to −65°C and 23.0 g (0.172 mole) of ethyl 2-methylthioacetate were added. Immediately, 18.9 g (0.175 mole) of t-butylhypochlorite were added dropwise. After the addition was completed, the mixture was stirred at −65°C for 1.5 hours, treated with 17.2 g (0.125 mole) of triethylamine and allowed to warm up to ambient temperature. The methylene chloride solution was washed twice with two 100 ml portions of water, concentrated, and the residue dissolved in 200 ml of methanol. This solution was heated to reflux, treated with 40 ml of 3N hydrochloric acid and the mixture heated at reflux overnight. The dark solution was concentrated to approximately 100 ml, when a solid began to precipitate. The mixture was cooled, the solid collected by filtration, washed with a small volume of cold methanol and dried to give 22.8 g (52%) of yellow material. A nuclear magnetic resonance (NMR) analysis of the material indicated that the solid was a mixture of the 4-phenyl isomer and the 6-phenyl isomer in a ratio of 2:1. The 4-phenyl isomer was separated from the 6-phenyl isomer by fractional crystallization from benzene. Three recrystallizations of the yellow solid from benzene gave 8.5 g (19%) of 3-methylthio-4-phenylindolin-2-one as a white solid, m.p. 182—5°C.

| | | | | |
|---|---|---|---|---|
| Analysis: | Calculated for $C_{15}H_{13}NOS$: | C, 70.56; | H, 5.13; | N, 5.49 |
| | Found | C, 70.26, | H, 5.16; | N, 5.14 |

*Preparation2*

4-Phenylindolin-2-one

A stirred solution of 3.5 g (0.014 mole) of 4-phenyl-3-methylthioindolin-2-one in 75 ml of tetrahydrofuran was treated portionwise with 20 g of a commercial Raney nickel/water suspension. The mixture was filtered through Celite (Registered Trade Mark) and the filtrate concentrated to give a yellow solid. This solid was recrystallized from benzene to yield 1.6 g (57%) of product as an off-white solid. An analytical sample was prepared from isopropanol; m.p. 192—194°C.

| | | | | |
|---|---|---|---|---|
| Analysis: | Calculated for $C_{14}H_{11}NO$: | C, 80.36; | H, 5.30; | N, 6.69 |
| | Found | C, 80.52; | H, 5.41; | N, 6.72 |

*Preparation 3*

3-Methylthio-7-methyl-4-phenylindolin-2-one

A solution of 30.6 g (0.13 mole) of 3-amino-4-methylbiphenyl in 400 ml of methylene chloride was cooled to −65°C and treated with 17.0 g (0.13 mole) of ethyl 2-methylthioacetate and then 14.2 g (0.132 mole) of t-butylhypochlorite while maintaining the temperature below −60°C. The reaction mixture was stirred at −65°C for 1.5 hours, treated with 13.3 (0.132 mole) of triethyl amine and allowed to warm up to ambient temperature. The solution was washed twice with water and concentrated. The residue was dissolved in 200 ml of methanol, 40 ml of 3N hydrochloric acid were added, and the mixture refluxed overnight. The solution was concentrated until a solid began to precipitate. The mixture was cooled, and the solid collected by filtration.

*Preparations 4A to 4I*

When in the procedure of Preparation 3 and in the manner of the preceding discussion, 3-amino-4-methylbiphenyl was replaced by equal molar amounts of the following:

3-amino-5-methylbiphenyl,
3-amino-2′-methoxybiphenyl,
3-amino-2′,5′dimethoxybiphenyl,
3-amino-3′-chlorobiphenyl,
3-amino-3′,5′-dibromobiphenyl,
3-amino-4-chlorobiphenyl,
3-amino-4,6-dichlorobiphenyl,
3-amino-6-methyl-2′-methylbiphenyl,
3-amino-2′,4-dichlorobiphenyl,

there were obtained

3 - methylthio - 6 - methyl - 4 - phenylindolin - 2 - one and 3 - methylthio - 4 - methyl - 6 - phenylindolin - 2 - one, (4A)

3 - methylthio - 4 - (2 - methoxyphenyl)indolin - 2 - one and 3 - methylthio - 6 - (2 - methoxyphenyl)indolin - 2 - one, (4B)

3 - methylthio - 4 - (2,5 - dimethoxyphenyl)indolin - 2 - one and 3 - methylthio - 6 - (2,5 - dimethoxyphenyl)indolin - 2 - one, (4C)

3 - methylthio - 4 - (3 - chlorophenyl)indolin - 2 - one and 3 - methylthio - 6 - (3 - chlorophenyl)indolin - 2 - one, (4D)

3 - methylthio - 4 - (3,5 - dibromophenyl)indolin - 2 - one and 3 - methylthio - 6 - (3,5 - dibromophenyl)indolin - 2 - one, (4E)

3 - methylthio - 7 - chloro - 4 - phenylindolin - 2 - one, (4F)

3 - methylthio - 5,7 - dichloro - 4 - phenylindolin - 2 - one, (4G)

3 - methylthio - 5 - methyl - 4 - (2 - methylphenyl)indoline - 2 - one (4H) and 3 - methylthio - 7 - chloro - 4 - (2 - chlorophenyl)indolin - 2 - one. (4J).

*Preparation 5*

6-Methyl-4-phenylindolin-2-one

To a slurry of 11.8 g (0.044 mole) of 3-methylthio-6-methyl-4-phenylindoline-2-one in 500 ml of tetrahydrofuran were added 100 g of a commercial Raney nickel/water preparation portionwise over a two-hour period. The mixture was filtered through Celite (Registered Trade Mark) and the filtrate concentrated. A small amount of methylene chloride was added to the residue and the resulting solid was collected by filtration.

*Preparations 6A to 6J*

When in the procedure of Preparation 5 and in the manner of the preceding discussion, 3-methylthio-6-methyl-4-phenylindolin-2-one was replaced by equal molar amounts of the following methylthioindolin-2-ones:

3-methylthio-7-methyl-4-phenylindolin-2-one,
3-methylthio-6-methyl-4-phenylindolin-2-one,
3-methylthio-4-(2-methoxyphenyl)indolin-2-one,
3-methylthio-4-(2,5-dimethoxyphenyl)indolin-2-one,
3-methylthio-4-(3-chlorophenyl)indolin-2-one,
3-methylthio-4-(3,5-dibromophenyl)indolin-2-one,
3-methylthio-7-chloro-4-phenylindolin-2-one,
3-methylthio-5,7-dichloro-4-phenylindolin-2-one,
3-methylthio-4-(2-methylphenyl)indolin-2-one, and
3-methylthio-7-chloro-4-(2-chlorophenyl)indolin-2-one,

there were obtained

7-methyl-4-phenylindolin-2-one, (6A)
6-methyl-4-phenylindolin-2-one, (6B)
4-(2-methoxyphenyl)indolin-2-one, (6C)
4-(2,5-dimethoxyphenyl)indolin-2-one, (6D)
4-(3-chlorophenyl)indolin-2-one, (6E)
4-(3,5-dibromophenyl)indolin-2-one, (6F)
7-chloro-4-phenylindolin-2-one, (6G)
5,7-dichloro-4-phenylindolin-2-one, (6H)
5-methyl-4-(2-methylphenyl)indolin-2-one, (6I) and
7-chloro-4-(2-chlorophenyl)indolin-2-one. (6J)

*Preparations 7A to 7C*

When in the procedure of Preparation 3 and in the manner of the preceding discussion, 3-amino-4-methylbiphenyl was replaced by equal molar amounts of the following nitro derivatives:

3-amino-3′-nitrobiphenyl,
3-amino-2′-nitrobiphenyl,
3-amino-4,6-dinitrobiphenyl,

there were obtained.

3 - methylthio - 4 - (3 - nitrophenyl)indolin - 2 - one, and 3 - methylthio - 6 - (3 - nitrophenyl)indolin - 2 - one, (7A)

3 - methylthio - 6 - (3 - nitrophenyl)indolin - 2 - one, and 3 - methylthio - 6 - (2 - nitrophenyl)indolin - 2 - one, (7B) and

3 - methylthio - 4 - phenyl - 5,7 - dinitroindolin - 2 - one (7C)

*Preparation 8*

4-Phenyl-5,7-diaminoindolin-2-one

A mixture of 4.9 g (0.014 mole) of 3-methylthio-4-phenyl-5,7-dinitroindolin-2-one (see Preparation 7C), 4.2 g (0.035 mole) of tin powder, 10 ml of concentrated hydrochloric acid and 50 ml of ethanol were refluxed for 4 hours under nitrogen. The hot mixture was filtered and the filtrate concentrated to give a solid.

*Preparations 9A and 9B*

When in the procedure of Preparation 8 and in the manner of the preceding discussion, 3-methylthio-4-phenyl-5,7-dinitroindolin-2-one was replaced by equal molar amounts of the following nitro-methylthio-indolins of Preparation 7:

3-methylthio-4-(3-nitrophenyl)indolin-2-one, (see 7A)
3-methylthio-4-(2-nitrophenyl)indolin-2-one, (see 7B)
there were obtained
4-(3-aminophenyl)indolin-2-one, (9A) and
4-(2-aminophenyl)indolin-2-one (9B).

Example 1

2-Amino-3-methyl-6-biphenylacetic Acid Hydrate (1:4)

A mixture of 4.79 g (0.0215 mole) of 7-methyl-4-phenylindolin-2-one (see Preparation 6A) and 60 ml of 3N sodium hydroxide was heated at reflux under nitrogen for 17 hours. The reaction mixture was cooled, filtered, and the filtrate diluted with 60 ml of water. The solution was cooled, made acidic with glacial acid and the resulting solid immediately collected by filtration, washed with cold water and dried under vacuum.

Example 2

2-Amino-6-biphenylacetic Acid

A mixture of 1.5 g (0.007 mole) of 4-phenylindolin-2-one (see Preparation 2) and 20 ml of 3N sodium hydroxide was heated at reflux for 6 hours. The mixture was cooled and filtered. The filtrate was made acidic with glacial acetic acid, and the resulting solid collected by filtration, washed with water and dried to yield 0.5 g (32%) of product as a tan powder, m.p. 190—191°C (dec.).

| | | | |
|---|---|---|---|
| Analysis: Calculated for $C_{14}H_{13}NO_2$: | C, 73,99; | H, 5.77: | N, 6.16 |
| Found | C, 73.54; | H, 5.71; | N, 6.28 |

Example 3

Sodium 2-amino-6-biphenylacetate

A solution of 2.8 g (0.0125 mole) of crude 2-amino-6-biphenylacetic acid in 40 ml of tetrahydrofuran was treated with 2 ml of 5% sodium hydroxide. The solution was concentrated and the residue subject to a benzene azetrope to eliminate water. The resulting tan solid was recrystallized three times from ethyl alcohol to give a pure sample of the sodium salt as a tan solid, m.p. 128—48°C. (dec.)

| | | | |
|---|---|---|---|
| Analysis: Calculated for $C_{14}H_{12}NaNO_2$: | C, 67.46; | H, 4.85; | N, 5.62 |
| Found | C, 67.25; | H, 4.96; | N, 5.65 |

Example 4

When in the procedure of Example 1 and in the manner of the preceding discussion, 7-methyl-4-phenylindolin-2-one was replaced by equal molar amounts of
5-methyl-4-(2-methylphenyl)indolin-2-one (see 6I)
there was obtained
2-amino-5,2′-dimethyl-6-biphenylacetic acid.

Examples 5A and 5B

When in the procedure of Example 1 and in the manner of the preceding discussion, 7-methyl-4-phenylindolin-2-one was replaced by equal molar amounts of
4-(2-methoxyphenyl)indolin-2-one, (see 6C)
4-(2,5-dimethoxyphenyl)indolin-2-one (see 6D)
there were obtained
2-amino-2′-methoxy-6-biphenylacetic acid (Example 5A)
2-amino-2′,5′-dimethoxy-6-biphenylacetic acid (Example 5B)

Examples 6A and 6B

When in the manner of Example 1 and in the manner of the preceding discussion, 7-methyl-4-phenylindolin-2-one was replaced by equal molar amounts of
4-(3,5-dibromophenyl)indolin-2-one, (see 6F)
7-chloro-4-(2-chlorophenyl)indolin-2-one, (see 6J)
there were obtained
2-amino-3′,5′-dibromo-6-biphenylacetic acid, (Example 6A)
2-amino-2′,3-dichloro-6-biphenylacetic acid (Example 6B)

Examples 7A and 7B

When in the procedure of Example 1, 2 moles of potassium hydroxide per mole of the indolin-2-one was substituted for the sodium hydroxide, ethanol was substituted for water and 7-methyl-4-phenylindolin-2-one was replaced by equal molar amounts of
4-(3-aminophenyl)indolin-2-one, (see 9A)
4-(2-aminophenyl)indolin-2-one (see 9B)
there were obtained

2,3'-diamino-6-biphenylacetic acid (Example 7A)
2,2'-diamino-6-biphenylacetic acid (Example 7B)

Example 8

When in the procedure of Example 1, 2 moles of potassium hydroxide per mole of the phenylindolin-2-one was substituted for the sodium hydroxide, ethanol was substituted for water and 7-methyl-4-phenylindolin-2-one was replaced by equal molar amounts of
4-phenyl-5,7-diaminoindolin-2-one (see 8)
there was obtained
2,3,5-triamino-6-biphenylacetic acid.

Examples 9A to 9D

When in the procedure of Example 1 and in the manner of the preceding discussion, 7-methyl-4-phenylindolin-2-one was replaced by equal molar amounts of
6-methyl-4-phenylindolin-2-one (see 6B)
4-(3-chlorophenyl)indolin-2-one (see 6E)
7-chloro-4-phenylindolin-2-one (see 6G)
5,7-dichloro-4-phenylindolin-2-one, (see 6H)
there were obtained
2-amino-4-methyl-6-biphenylacetic acid (Example 9A)
2-amino-3'-chloro-6-biphenylacetic acid (Example 9B)
2-amino-3-chloro-6-biphenylacetic acid (Example 9C)
2-amino-3,5-dichloro-6-biphenylacetic acid (Example 9D).

Example 10

Ethyl 2-Amino-6-biphenylacetate

Sodium 2-amino-6-biphenylacetate was dissolved in dimethylformamide and the solution treated with ethyliodide. The solution was stirred at room temperature for about 3 hours, the solution added to water and the mixture extracted several times with benzene. The combined benzene extracts were washed with dilute base and water, dried over sodium sulphate, concentrated under reduced pressure and crystallized to give ethyl 2-amino-6-biphenylacetate.

Formulation and Administration

The present invention also contemplates compositions containing the compounds of the present invention as active ingredients. Effective quantities of any of the foregoing pharmacologically active compounds may be administered to a living animal body in any one of various ways. For example, they may be orally administered in the form of sterile solutions. In forming the compositions of this invention, the active ingredient is incorporated in a suitable carrier such as a pharmaceutical carrier. Suitable pharmaceutical carriers which are useful in formulating the compositions of this invention include starch, gelatin, glucose, magnesium carbonate, lactose, and malt. Liquid compositions are also within the purview of this invention and suitable liquid pharmaceutical carriers include ethyl alcohol, propylene glycol, glycerine, and glucose syrup.

The pharmacologically active compounds of the present invention may be advantageously employed in a unit dosage of from about 1 to about 100 milligrams. The unit dosage may be given a suitable number of times daily so that the daily dosage may vary from 1 to 500 milligrams. Five to 50 milligrams appears to be an optimum unit dose.

It is only necessary that the active ingredient constitute an effective amount; i.e. such that a suitable effective dosage will be obtained consistent with the dosage form employed. The exact individual dosages as well as daily dosages will, of course, be determined according to standard medical principles under the direction of a physician or veterinarian.

The active agents of the present invention may be combined with the pharmacologically active agents, or with buffers, antacids or the like for administration. The proportion of the active agent in the compositions may be varied widely.

The following are examples of compositions formed in accordance with this invention.

Examples 11A to 11C

Capsules of 5 mg, (Example 11A), 25 mg (Example 11B), and 50 mg (Example 11C) of active ingredient per capsule were prepared. With the higher amounts of active ingredient, adjustment may be made in the amount of lactose.

Example 11A

| Typical blend for encapsulation | | Per Capsule mg |
|---|---|---|
| Active ingredient | | 5.0 |
| Lactose | | 296.7 |
| Starch | | 129.0 |
| Magnesium stearate | | 4.3 |
| | TOTAL | 435.0 mg |

Example 11B

Additional capsule formulations preferably contain a higher dosage of active ingredient and are as follows:—

| Ingredients | | Per Capsule mg |
|---|---|---|
| Active ingredient | | 25.0 |
| Lactose | | 306.5 |
| Starch | | 99.2 |
| Magnesium stearate | | 4.3 |
| | TOTAL | 435.0 mg |

In each case the selected active ingredient was uniformly blended with the lactose, starch, and magnesium stearate and the blend encapsulated.

Example 12

A typical formulation for a tablet containing 5.0 mg of active ingredient per tablet is as follows. The formulation may be used for other strengths of active ingredient by adjusting the weight of the dicalcium phosphate.

| | Per tablet, mg |
|---|---|
| (1) Active ingredient | 5.0 |
| (2) Corn starch | 13.6 |
| (3) Corn starch (paste) | 3.4 |
| (4) Lactose | 79.2 |
| (5) Dicalcium phosphate | 68.0 |
| (6) Calcium stearate | 0.9 |
| | 170.1 mg |

The tablets were formed by uniformly blending ingredients 1, 2, 4 and 5. Ingredient 3 was prepared as a 10 percent paste in water. The blend was granulated with starch paste and the resulting wet mass passed through an eight mesh screen (U.S. Sieve size), having openings of 2.4 mms. The wet granulations was dried and sized through a twelve mesh screen (U.S. Sieve size) having openings of 1.7 mms. The dried granules were blended with the calcium stearate and pressed.

Example 13

An injectable 2% sterile solution was made up as follows:—

| | Per cc |
|---|---|
| Active ingredient | 20 mg |
| Preservative, e.g. chlorobutanol | 0.5% weight/volume |
| Water for injection | q.s. |

The solution was prepared, clarified by filtration and placed into vials. The vials were sealed and heated in an autoclave.

**Claims for the Contracting States: BE CH DE FR GB IT LI LU NL SE**

1. A compound having the structural formula:—

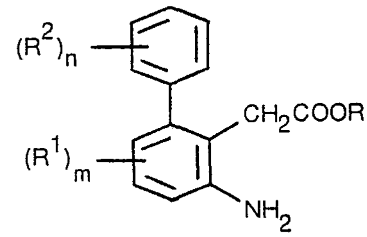

wherein:

R represents a hydrogen atom or a lower alkyl group

$R^1$ represents a fluorine, chlorine or bromine atom or a lower alkyl or amino group.

$R^2$ represents a lower alkyl, lower alkoxy, amino or trifluoromethyl group or a fluorine, chlorine or bromine atom,

n is 0—3 and m is 0—2,

or a pharmaceutically acceptable salt thereof or a hydrate thereof.

2. 2-Amino-6-biphenylacetic acid or a pharmaceutically acceptable salt thereof.

3. A compound as claimed in claim 1 in which $R^1$ represents a methyl group or an amino group or a chlorine atom, and m = 1, and n = 0.

4. A compound as claimed in claim 1 in which m = 0, $R^2$ represents a methoxy group, an amino group, or a chlorine atom and n = 1.

5. A compound as claimed in claim 1 in which m = 2, $R^1$ represents an amino group or a chlorine atom and n = 0.

6. A compound as claimed in claim 1 in which m = 0, $R^2$ represents a methoxy group or a bromine atom and n = 2.

7. A compound as claimed in claim 5 or claim 6 in which the $R^1$ substituents are the same or the $R^2$ substituents are the same.

8. A muscle relaxation or inflammation alleviating composition comprising (a) as the active ingredient an effective amount of a compound as claimed in anyone of claims 1 to 7 and (b) a pharmaceutically acceptable carrier therefor.

9. A composition as claimed in claim 8 in which the effective amount ranges between 1 and 100 milligrams of the active ingredient.

10. A compound as claimed in anyone of claims 1 to 7 or a composition as claimed in claim 8 or claim 9 for use in muscle relaxation in a living animal body or for use in alleviating inflammation in a living animal body.

**Claims for the Contracting State: AT**

1. A process for the preparation of a compound of general formula I,

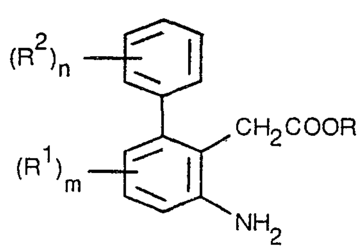

Formula I

wherein:

R represents a hydrogen atom or a lower alkyl group,

$R^1$ represents a fluorine, chlorine or bromine atom or a lower alkyl or amino group,

$R^2$ represents a lower alkyl, lower alkoxy, amino or trifluoromethyl group or a fluorine, chlorine or bromine atom,

n is 0—3 and m is 0—2,

or a pharmaceutically acceptable salt or hydrate thereof,

the process comprising (i) hydrolysing a compound of general formula II:

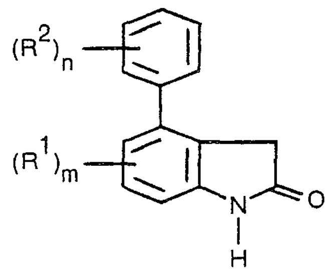

(II)

wherein $R^1$, $R^2$, n and m are as defined for general formula I above, in aqueous basic solution followed by neutralisation of the basic reaction mixture with a suitable organic acid or dilute mineral acid to form a compound of general formula I in which R represents a hydrogen atom, and then (ii) optionally, to form a compound of general formula I in which R represents a lower alkyl group, converting the acid formed in step (i) to an alkali metal salt and reacting the alkali metal salt so formed with a lower alkyl halide in a suitable solvent, and optionally after step (i) or step (ii) forming a pharmaceutically acceptable salt or hydrate of a compound of general formula I.

2. A process for the preparation of 2-amino-6-biphenylacetic acid, or a pharmaceutically acceptable salt thereof, the process comprising hydrolysing 4-phenylindoline-2-one in an aqueous basic solution and neutralising the basic reaction mixture with a suitable organic acid or dilute mineral acid, and optionally forming a pharmaceutically acceptable salt of 2-amino-6-biphenylacetic acid.

3. A process as claimed in Claim 1 in which, in the general formulae, $R^1$ represents a methyl group or an amino group or a chlorine atom, and m = 1, and n = 0.

4. A process as claimed in Claim 1 in which, in the general formulae, m = 0, $R^2$ represents a methoxy group, and amino group, or a chlorine atom and n = 1.

5. A process as claimed in Claim 1 in which, in the general formulae, m = 2, $R^1$ represents an amino group or a chlorine atom and n = 0.

6. A process as claimed in Claim 1 in which, in the general formulae, m = 0, $R^2$ represents a methoxy group or a bromine atom and n = 2.

7. A process as claimed in Claim 5 or Claim 6 in which, in the general formulae, the $R^1$ substituents are the same or the $R^2$ substituents are the same.

8. A process for the preparation of a muscle relaxation or inflammation alleviating composition comprising admixing (a) as the active ingredient an effective amount of a compound having the structural formula:—

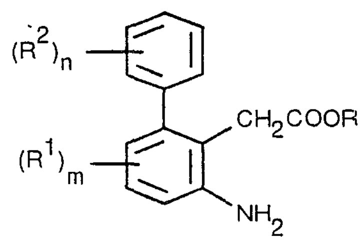

wherein:

R represents a hydrogen atom or a lower alkyl group,

$R^1$ represents a fluorine, chlorine or bromine atom or a lower alkyl or amino group,

$R^2$ represents a lower alkyl, lower alkoxy, amino or trifluoromethyl group or a fluorine, chlorine or bromine atom,

n is 0—3 and m is 0—2,

or a pharmaceutically acceptable salt thereof or a hydrate thereof,

and (b) a pharmaceutically acceptable carrier therefor.

9. A process as claimed in Claim 8 in which the effective amount ranges between 1 and 100 milligrams of the active ingredient.

**Patentansprüche für die Vertragsstaaten: BE CH DE FR GB IT LI LU NL SE**

1. Verbindung mit der Strukturformel

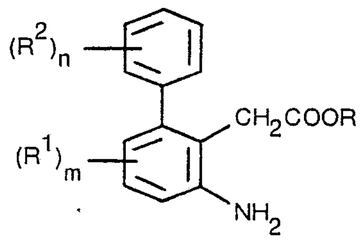

worin
R ein Wasserstoffatom oder eine Niederalkylgruppe bedeutet,
$R^1$ für ein Fluor-, Chlor- oder Bromatom oder für eine Niederalkyl- oder Aminogruppe steht,
$R^2$ eine Niederalkyl-, Niederalkoxy-, Amino- oder Trifluormethylgruppe oder ein Fluor-, Chlor- oder Bromatom darstellt,
n gleich 0 bis 3 und m gleich 0 bis 2 sind;
oder ein pharmazeutisch annehmbares Salz oder ein Hydrat davon.

2. 2-Amino-6-biphenylessigsäure oder ein pharmazeutisch annehmbares Salz davon.

3. Verbindung nach Anspruch 1, worin $R^1$ eine Methylgruppe oder eine Aminogruppe oder ein Chloratom bedeutet und m gleich 1 sowie n gleich 0 sind.

4. Verbindung nach Anspruch 1, worin m gleich 0 ist, $R^2$ eine Methoxygruppe, eine Aminogruppe oder ein Chloratom bedeutet und n gleich 1 ist.

5. Verbindung nach Anspruch 1, worin m gleich 2 ist, $R^1$ eine Aminogruppe oder ein Chloratom bedeutet und n gleich 0 ist.

6. Verbindung nach Anspruch 1, worin m gleich 0 ist, $R^2$ eine Methoxygruppe oder ein Bromatom bedeutet und n gleich 2 ist.

7. Verbindung nach Anspruch 5 oder 6, worin die Substituenten $R^1$ gleich sind oder die Substituenten $R^2$ gleich sind.

8. Zusammensetzung zur Muskelentspannung oder zur Abschwächung von Entzündung, enthaltend (a) als aktiven Bestandteil eine wirksame Menge einer Verbindung nach einem der Ansprüche 1 bis 7 und (b) einen pharmazeutisch annehmbaren Träger hiefür.

9. Zusammensetzung nach Anspruch 8, worin die wirksame Menge zwischen 1 und 100 mg des aktiven Bestandteiles beträgt.

10. Verbindung nach einem der Ansprüche 1 bis 7 oder Zusammensetzung nach Anspruch 8 oder 9 zur Verwendung für die Muskelentspannung in einem lebenden Tierkörper oder zur Verwendung für die Abschwächung von Entzündung in einem lebenden Tierkörper.

**Patentansprüche für den Vertragsstaat: AT**

1. Verfahren zur Herstellung einer Verbindung der allgemeinen Formel

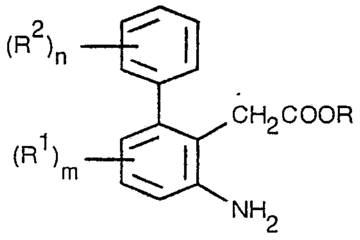

I

worin
R ein Wasserstoffatom oder eine Niederalkylgruppe bedeutet,
$R^1$ für ein Fluor-, Chlor- oder Bromatom oder für eine Niederalkyl- oder Aminogruppe steht,
$R^2$ eine Niederalkyl-, Niederalkoxy-, Amino- oder Trifluormethylgruppe oder ein Fluor-, Chlor- oder Bromatom darstellt,
n gleich 0 bis 3 und m gleich 0 bis 2 sind;
oder ein pharmazeutisch annehmbares Salz oder ein Hydrat davon,
dadurch gekennzeichnet, daß
(i) eine Verbindung der allgemeinen Formel

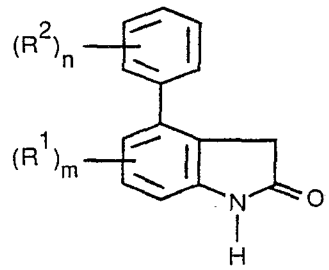

(II)

worin $R^1$, $R^2$, n und m wie vorstehend für die allgemeine Formel (I) definiert sind, in einer wässerigen, basischen Lösung hydrolysiert wird, gefolgt von einer Neutralisation des basischen Reaktionsgemisches mit einer geeigneten organischen Säure oder einer verdünnten Mineralsäure, um eine Verbindung der allgemeinen Formel (I) zu bilden, worin R ein Wasserstoffatom bedeutet, und daß dann

ii) gewünschtenfalls zur Bildung einer Verbindung der allgemeinen Formel (I), worin R eine Niederalkylgruppe bedeutet, die in Stufe (i) gebildete Säure in ein Alkalimetallsalz übergeführt wird und das so entstandene Alkalimetallsalz mit einem Niederalkylhalogenid in einem geeigneten Lösungsmittel umgesetzt wird, und gegebenenfalls nach Stufe (i) oder Stufe (ii) ein pharmazeutisch annehmbares Salz oder Hydrat einer Verbindung der allgemeinen Formel (I) gebildet wird.

2. Verfahren zur Herstellung der 2-Amino-6-biphenylessigsäure oder eines pharmazeutisch annehmbaren Salzes davon, dadurch gekennzeichnet, daß das 4-Phenylindolin-2-on in einer wässerigen, basischen Lösung hydrolysiert wird und das basische Reaktionsgemisch mit einer geeigneten organischen Säure oder verdünnten Mineralsäure neutralisiert wird und daß gewünschtenfalls ein pharmazeutisch annehmbares Salz der 2-Amino-6-biphenylessigsäure gebildet wird.

3. Verfahren nach Anspruch 1, worin in der allgemeinen Formel $R^1$ eine Methylgruppe oder eine Aminogruppe oder ein Chloratom bedeutet und m gleich 1 und n gleich 0 sind.

4. Verfahren nach Anspruch 1, worin in der allgemeinen Formel m gleich 0 ist, $R^2$ eine Methoxygruppe, eine Aminogruppe oder ein Chloratom bedeutet und n gleich 1 ist.

5. Verfahren nach Anspruch 1, worin in der allgemeinen Formel m gleich 2 ist, $R^1$ eine Aminogruppe oder ein Chloratom bedeutet und n gleich 0 ist.

6. Verfahren nach Anspruch 1, worin in der allgemeinen Formel m gleich 0 ist, $R^2$ eine Methoxygruppe oder ein Bromatom bedeutet und n gleich 2 ist.

7. Verfahren nach Anspruch 5 oder 6, worin in der allgemeinen Formel die Substituenten $R^1$ gleich sind oder die Substituenten $R^2$ gleich sind.

8. Verfahren zur Herstellung einer Zusammensetzung zur Muskelentspannung oder zur Abschwächung von Entzündungen, dadurch gekennzeichnet, daß

(a) als aktiver Bestandteil eine wirksame Menge einer Verbindung der Formel

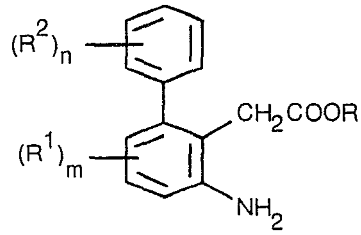

worin

R ein Wasserstoffatom oder eine Niederalkylgruppe bedeutet,

$R^1$ für ein Fluor-, Chlor- oder Bromatom oder für eine Niederalkyl- oder Aminogruppe steht,

$R^2$ eine Niederalkyl-, Niederalkoxy-, Amino- oder Trifluormethylgruppe oder ein Fluor-, Chlor- oder Bromatom darstellt,

n gleich 0 bis 3 und m gleich 0 bis 2 sind;

oder ein pharmazeutisch annehmbares Salz oder ein Hydrat davon und

(b) ein pharmazeutisch annehmbarer Träger hiefür gemischt werden.

9. Verfahren nach Anspruch 8, worin die wirksame Menge zwischen 1 und 100 mg des aktiven Bestandteiles beträgt.

**Revendications pour les Etats contractants: BE CH DE FR GB IT LI LU NL SE**

1. Composé de formule:

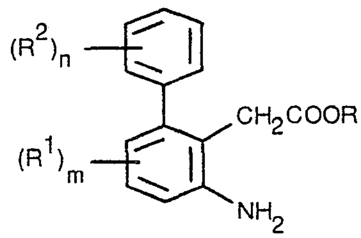

où

R représente un atome d'hydrogène ou un radical alcoyle inférieur,

$R^1$ représente un atome de fluor, de chlore ou de brome ou un radical alcoyle inférieur ou radical amino,

$R^2$ représente un radical alcoyle inférieur, alcoxy inférieur, amino ou trifluorométhyle ou un atome de fluor, de chlore ou de brome,

n représente 0 à 3 et m représente 0 à 2,

et les sels pharmaceutiquement acceptables de celui-ci ou un hydrate de celui-ci.

2. L'acide 2-amino-6-biphénylacétique ou un sel pharmaceutiquement acceptable de celui-ci.

3. Composé suivant la revendication 1, dans lequel $R^1$ représente un radical méthyle ou amino ou un atome de chlore et m = 1 et n = 0.

4. Composé suivant la revendication 1, dans lequel m = 0, $R^2$ représente un radical méthoxy ou amino ou un atome de chlore et n = 1.

5. Composé suivant la revendication 1, dans lequel m = 2, $R^1$ représente un radical amino ou un atome de chlore et n = 0.

6. Composé suivant la revendication 1, dans lequel m = 0, $R^2$ représente un radical méthoxy ou un atome de brome et n = 2.

7. Composé suivant la revendication 5 ou 6, dans lequel les substituants $R^1$ sont identiques et les substituants $R^2$ sont identiques.

8. Composition pour la relaxation musculaire ou le soulagement de l'inflammation, comprenant (a) comme constituant actif une quantité efficace d'un composé suivant l'une quelconque des revendications 1 à 7 et (b) un excipient pharmaceutiquement acceptable pour celui-ci.

9. Composition suivant la revendication 8, dans laquelle la quantité efficace s'échelonne de 1 à 100 mg de constituant actif.

10. Composé suivant l'une quelconque des revendications 1 à 7 ou composition suivant la revendication 8 ou 9 à utiliser pour la relaxation musculaire dans l'organisme d'un animal vivant ou pour soulager l'inflammation dans l'organisme d'un animal vivant.

**Revendications pour l'Etat contractant: AT**

1. Procédé de préparation d'un composé de formule générale I

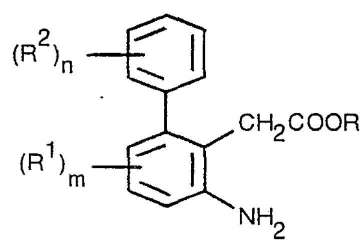

I

où

R représente un atome d'hydrogène ou un radical alcoyle inférieur,

$R^1$ représente un atome de fluor, de chlore ou de brome ou un radical alcoyle inférieur ou radical amino,

$R^2$ représente un radical alcoyle inférieur, alcoxy inférieur, amino ou trifluorométhyle ou un atome de fluor, de chlore ou de brome,

n représente 0 à 3 et m représente 0 à 2,

ou d'un sel pharmaceutiquement acceptable de celui-ci ou d'un hydrate de celui-ci,

le procédé comprenant

(i) l'hydrolyse d'un composé de formule générale II

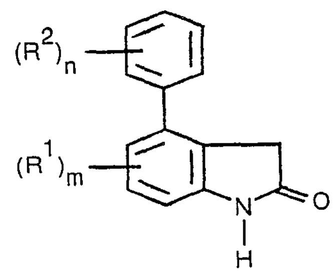

(II)

où $R^1$, $R^2$, n et m sont tels que définis à propos de la formule générale I ci-dessus, en solution aqueuse basique, suivie de la neutralisation du mélange de réaction basique a l'aide d'un acide organique approprié ou d'un acide minéral dilué pour former un composé de formule générale I où R représente un atome d'hydrogène et ensuite

(ii) éventuellement, pour former un composé de formule générale I où R représente un radical alcoyle inférieur, la conversion de l'acide formé au stade (i) en un sel de métal alcalin et la réaction du sel de métal alcalin ainsi obtenu avec un halogénure d'alcoyle inférieur dans un solvant approprié, et éventuellement après de stade (i) ou le stade (ii), la formation d'un sel pharmaceutiquement acceptable ou hydrate d'un composé de formule générale I.

2. Procédé de préparation de l'acide 2-amino-6-biphénylacétique, ou d'un sel pharmaceutiquement acceptable de celui-ci, le procédé comprenant l'hydrolyse de la 4-phénylindoline-2-one dans une solution aqueuse basique et la neutralisation du mélange de réaction basique à l'aide d'un acide organique approprié ou d'un acide minéral dilué, et éventuellement la formation d'un sel pharmaceutiquement acceptable de l'acide 2-amino-6-biphénylacétique.

3. Procédé suivant la revendication 1, dans lequel, dans les formules générales, $R^1$ représente un radical méthyle ou amino ou un atome de chlore, et m = 1 et n = 0.

4. Procédé suivant la revendication 1, dans lequel, dans les formules générales, m = 0, $R^2$ représente un radical méthoxy ou amino ou un atome de chlore et n = 1.

5. Procédé suivant la revendication 1, dans lequel, dans les formules générales, m = 2, $R^1$ représente un radical amino ou un atome de chlore et n = 0.

6. Procédé suivant la revendication 1, dans lequel, dans les formules générales, m = 0, $R^2$ représente un radical méthoxy ou un atome de brome et n = 2.

7. Procédé suivant la revendication 5 ou 6, dans lequel, dans les formules générales, les substituants $R^1$ sont identiques et les substituants $R^2$ sont identiques.

8. Procédé de préparation d'une composition pour la relaxation musculaire ou le soulagement de l'inflammation, qui comprend le mélange (a) comme constituant actif, d'une quantité efficace d'un composé de la formule de structure

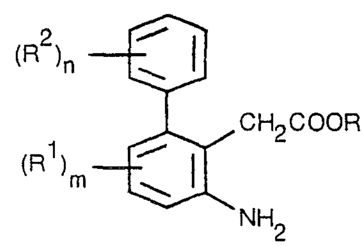

où

R représente un atome d'hydrogène ou un radical alcoyle inférieur,

$R^1$ représente un atome de fluor, de chlore ou de brome ou un radical alcoyle inférieur ou radical amino,

$R^2$ représente un radical alcoyle inférieur, alcoxy inférieur, amino ou trifluorométhyle ou un atome de fluor, de chlore ou de brome,

n représente 0 à 3 et m représente 0 à 2,

ou d'un sel pharmaceutiquement acceptable de celui-ci ou d'un hydrate de celui-ci et (b) d'un excipient pharmaceutiquement acceptable pour celui-ci.

9. Procédé suivant la revendication 8, dans lequel la quantité efficace s'échelonne de 1 à 100 mg du constituant actif.